# EUROPEAN PATENT APPLICATION

(11) **EP 1 570 791 A1**
(43) Date of publication of application: **07.09.2005**
(21) Application number: 05251315.7
(22) Date of filing: 04.03.2005
(51) Int. Cl.: A61B 17/17, A61B 17/16

(54) **Guidance system for rotary surgical instrument**

(30) Priority: 05.03.2004 US 794657
(71) Applicant: Zimmer Technology, Inc., Illinois 60606 (US)
(72) Inventor: Grimm, James E., Winona Lake, Indiana 46590 (US); Walriven, Dale, Warsaw, Indiana 46580 (US); McGinley, Shawn E., Fort Wayne, Indianan 46814 (US); Rangaiah, Chetan, Warsaw, Indiana 46582 (US)
(74) Representative: Mays, Julie

(57) **Abstract**

A surgical instrument for use in a computer assisted navigation system is provided. The instrument includes at least one reference element registerable in the navigational system disposed on a mounting assembly. The mounting assembly is rotatably mounted on the instrument and includes a counterweight or other anti-rotation feature whereby the reference element may be maintained in a desired orientation. A rotatable shaft may extend through the mounting assembly and have one end which is securable to a rotary driver such as a drill and a second end securable to a rotary tool such as a reamer. The second end of the shaft may utilize a collet assembly to firmly grasp the driven tool and thereby limit relative movement of the tool and the reference elements disposed on the mounting assembly.

## Description

### BACKGROUND OF THE INVENTION

This application is a Continuation-In-Part of Serial No. 10/357,592, filed on February 4, 2003.

### 1. Field of the Invention.

The present invention relates to computer assisted surgical navigation systems and, more specifically, to the use of a rotary instrument in a computer assisted surgical navigation system.

### 2. Description of the Related Art.

The controlled positioning of surgical instruments is of significant importance in many surgical procedures and various methods and guide instruments have been developed for properly positioning a surgical instrument. Such methods include the use of surgical guides which function as mechanical guides for aligning reamers, awls and other drilling and rotating instruments. The use of such surgical guides is common in orthopedic surgical procedures and such guides may be used to properly align a drill or other instrument with respect to a bone when preparing the bone for receiving an implant such as an artificial joint.

Computer assisted surgical navigation systems which provide for the image guidance of a surgical instrument are also known. Examples of various computer assisted navigation systems which are known in the art are described in United States Patent Nos. 5,682,886; 5,921,992; 6,096,050; 6,348,058 B1; 6,434,507 B1; 6,450,978 B1; 6,490,467 B1; and 6,491,699 B1, the disclosure of each of these patents is hereby incorporated herein by reference. Image guidance techniques typically involve acquiring preoperative images of the relevant anatomical structures and generating a data base which represents a three dimensional model of the anatomical structures. The relevant surgical instruments typically have a known and fixed geometry which is also defined preoperatively. During the surgical procedure, the position of the instrument being used is registered with the anatomical coordinate system and a graphical display showing the relative positions of the tool and anatomical structure may be computed in real time and displayed for the surgeon to assist the surgeon in properly positioning and manipulating the surgical instrument with respect to the relevant anatomical structure. It is also known in such computer assisted navigation systems to provide a guide for a rotary shaft that includes an array mounted on the guide for registering the guide in the coordinate system of the navigation system.

### SUMMARY OF THE INVENTION

The present invention provides a rotary surgical instrument which can be used with a computer assisted navigation system. A mounting assembly is provided that has at least one reference element registerable in the computer assisted navigation system. The mounting assembly is rotatable relative to the instrument. For example, a rotating shaft may extend through a cylindrical opening in the mounting assembly. The mounting assembly is biased so that the reference element is positioned in a desired orientation during operation of the instrument. For example, the mounting assembly may include a counterweight positioned opposite the reference element whereby the reference element is gravitationally biased toward a position above the rotational axis of the mounting assembly. This can be particularly useful in a computer assisted navigational system that requires the reference elements to be within the line of sight of the sensors tracking the movement of the reference elements, such as an optical tracking system.

The invention comprises, in one form thereof, a surgical instrument for use in a computer assisted navigation system. The instrument includes at least one reference element registerable in the computer assisted navigation system and a mounting assembly defining an axis and rotatably mounted on the instrument. The reference element is positionable on the mounting assembly in a predetermined location which defines a first angular position relative to the axis. A counterweight is disposed on the mounting assembly and is radially outwardly spaced from the axis. The counterweight defines a second angular position relative to the axis and the first and second angular positions are separated by at least 90 degrees.

The at least one reference element may take the form of at least three non-linearly positioned reference elements. The mounting assembly may also include a radially outwardly extending mounting stem that is disposed substantially diametrically opposite the counterweight relative to the axis with the at least one reference element being mountable on a radially distal end of the mounting stem. The mounting assembly may also include a sleeve portion defining a cylindrical opening with the counterweight being integrally formed with the sleeve portion. A rotary member having a cylindrical shaft portion may be rotationally engaged with the mounting assembly.

The invention may also include a locking assembly for temporarily locking the mounting assembly with respect to the rotary member, to prevent relative rotation therebetween. In the preferred embodiment, the locking assembly includes a shaft retainer positioned on the rotary member for rotation therewith and a locking lever that is pivotably attached to the mounting assembly. The locking lever is preferably configured to engage a portion of the shaft retainer, thereby preventing relative rotation between the rotary member and the mounting assembly.

Preferably, the locking assembly further includes one or more notches on either the shaft retainer or on the locking lever and a one or more projections on the other component, i.e., the component without the notch. The projection (or projections) is (are) configured to mate with the notch (or notches) for preventing relative rotation between the rotary member and the mounting assembly. The locking assembly also preferably includes spring member for biasing the locking lever into an unlocked position, i.e., out of engagement with the shaft retainer.

The invention comprises, in another form thereof, a surgical instrument for use in a computer assisted navigation system. The instrument includes a rotary member having first and second opposed ends and a mounting assembly operably coupled to the rotary member wherein the rotary member and the mounting assembly are relatively rotatable about an axis. At least one reference element registerable in the computer assisted navigation system is disposed on the mounting assembly at a predetermined location. The mounting assembly defines a center of gravity that is spaced radially outwardly from the axis. The predetermined location of the reference element defines a first angular position relative to the axis and the center of gravity defines a second angular position relative to the axis wherein the first and second angular positions are separated by at least 90 degrees.

The mounting assembly may include a sleeve portion defining a cylindrical opening with the rotary member rotationally disposed within the cylindrical opening. The mounting assembly has a counterweight portion disposed radially outwardly from the axis wherein the at least one reference element is disposed substantially diametrically opposite the counterweight portion relative to the axis.

A rotational driver may be detachably secured to the first end of the shaft and a rotatable tool detachably secured to the second end. A collet assembly may also be disposed at the second end. The collet assembly may include a collet and a biasing member wherein the collet defines a central void and has a plurality of fingers biasable inwardly relative to the void. The biasing member is biasingly engageable with the plurality of collet fingers and is securable in a position biasing the plurality of fingers inwardly relative to the central void. A surgical tool having a shank may be inserted into the central void wherein the shank is rotationally fixedly engageable by the inwardly biasable plurality of collet fingers. The second end of the shaft may be defined by a cylindrical shaft having exterior threads and an axially disposed opening. The collet is partially positioned in the opening and at least a portion of the plurality of collet fingers projects from the opening. The biasing member threadingly engages the exterior threads and circumscribes the projecting portion of the plurality of collet fingers.

The invention comprises, in yet another form thereof, a surgical instrument for use in a computer assisted navigation system. The instrument includes a rotary member and a mounting assembly operably coupled to the rotary member wherein the rotary member and the mounting assembly are relatively rotatable about an axis. At least one reference element registerable in the computer assisted navigation system is disposed on the mounting assembly at a predetermined location. An anti-rotation feature disposed on the mounting assembly biases the mounting assembly toward an orientation wherein the at least one reference element is disposed vertically above the axis during relative rotation of the rotary member and the mounting assembly with the axis being horizontally disposed. The anti-rotation feature may be a counterweight secured to the mounting assembly diametrically opposite the reference element relative to the axis.

The invention comprises, in still another form thereof, a method of providing a rotary surgical tool for use in a computer assisted navigation system. The method includes providing a shaft and coupling a mounting assembly with the shaft wherein the mounting assembly and the shaft are relatively rotatable about an axis. The mounting assembly has disposed thereon at least one reference element that is registerable in the computer assisted navigation system. The method also includes rotating the shaft relative to the mounting assembly and simultaneously non-manually biasing the mounting assembly toward a desired orientation relative to the axis wherein the at least one reference element is disposed vertically above the axis when the axis is oriented horizontally. The biasing of the mounting assembly toward a desired orientation may include disposing a counterweight on the mounting assembly and gravitationally biasing the reference element. The method may also include the step of coaxially securing a rotatable tool to the shaft with a collet assembly.

An advantage of the present invention is that it provides a means for mounting a reference element registrable in a computer assisted navigation system on a surgical instrument having a rotary member and maintaining the reference element in a desired orientation relative to the surrounding environment during operation of the tool. This can allow the reference element to be positioned generally above the tool to facilitate maintaining a line of sight between the reference element and a sensor. The ability to maintain the reference element within the line of sight of a navigation sensor is of particular importance for some types of computer assisted navigation systems, such as optical systems that detect light reflected from or generated by the reference elements.

Another advantage of the present invention is that it provides a collet assembly that allows the shank of a rotating tool to be firmly grasped and thereby limits any movement of the rotational axis of the tool relative to the at least one reference element which is used by the computer assisted navigational system to compute the position of the rotating tool.

### BRIEF DESCRIPTION OF THE DRAWINGS

The above mentioned and other features and objects of this invention, and the manner of attaining them, will become more apparent and the invention itself will be better understood by reference to the following description of embodiments of the invention taken in conjunction with the accompanying drawings, wherein:
Figure 1 is an exploded view of a surgical instrument in accordance with one embodiment of the present invention;
Figure 2 is an exploded, partially cross-sectional view of a rotary shaft and collet assembly of the Figure 1 embodiment;
Figure 3 is a cross-sectional view of a mounting member of the Figure 1 embodiment;
Figure 4 is a partially cross-sectional view of a quick-connect fitting, which is an alternative end configuration;
Figure 5 is an end view of the mounting member of the Figure 1 embodiment;
Figure 6 is an exploded view of a surgical instrument in accordance with another embodiment of the present invention;
Figure 7 is a partially exploded, partially cross-sectional view of the Figure 6 embodiment; and
Figure 8 is an end view of the locking assembly of the Figure 6 embodiment, which has been partially cut-away in order to better show some features of the assembly.

Corresponding reference characters indicate corresponding parts throughout the several views of the various embodiments. Although the exemplifications set out herein illustrate embodiments of the invention, the embodiments disclosed below are not intended to be exhaustive or to be construed as limiting the scope of the invention to the precise forms disclosed.

### DESCRIPTION OF THE PRESENT INVENTION

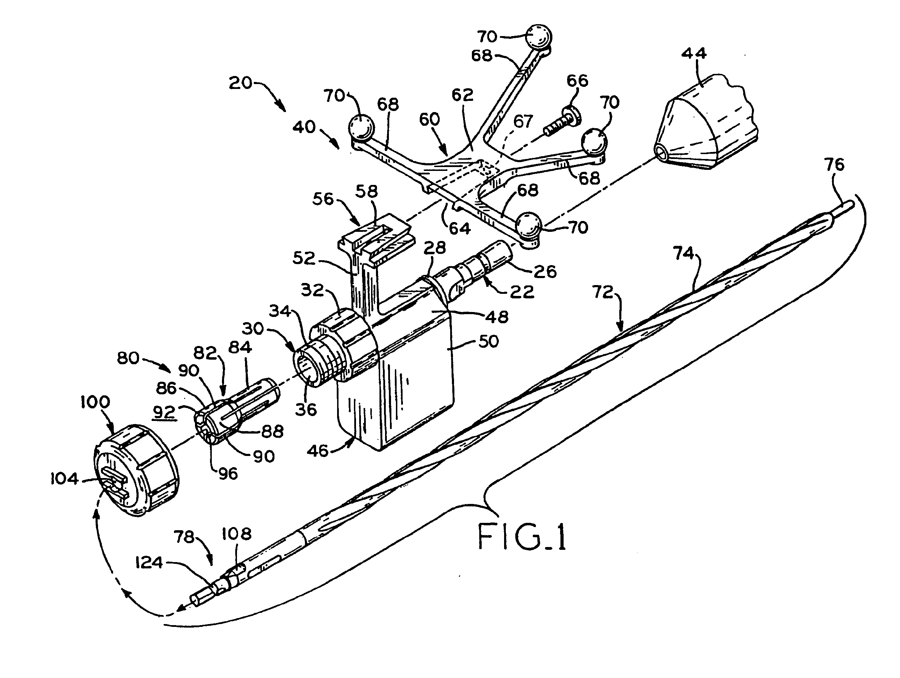

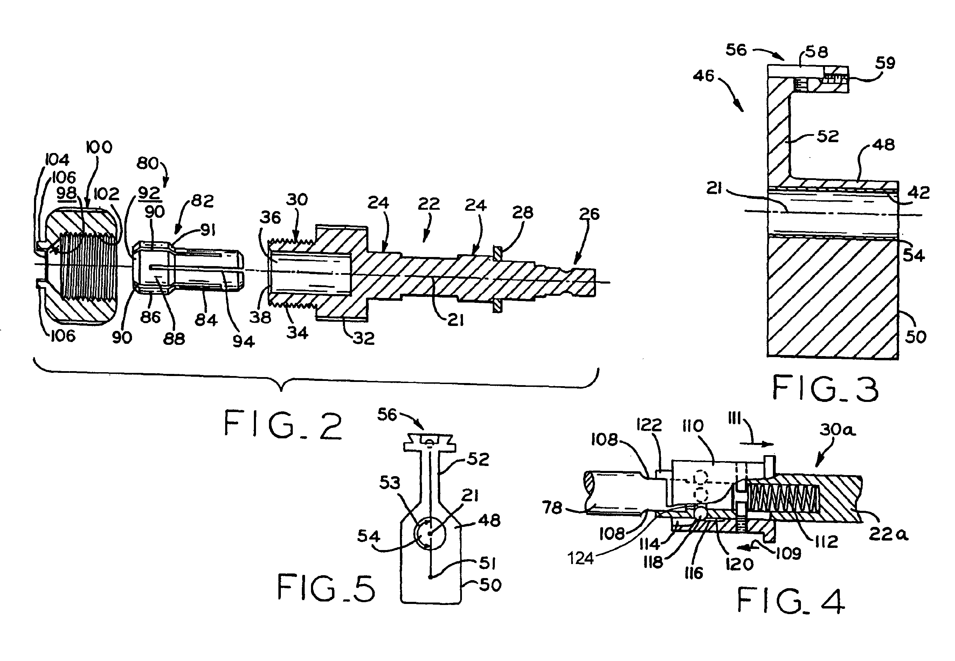

An exploded view of a surgical instrument 20 in accordance with one embodiment of the present invention is shown in Figure 1. Surgical instrument 20 includes a rotary member 22 which is rotationally engaged with mounting assembly 40. Rotary member 22 is best seen in Figures 1 and 2, and forms a shaft having two cylindrical portions 24 configured to be engaged with bearing sleeve insert 42 (Figure 3) located in mounting assembly 40. Rotary member (or shaft) 22 also includes a first end 26 which has a conventional shape for engagement with a surgical drill 44 or other powered or manual rotary driver. the rotary member 22 is preferably made of stainless steel, although other materials are also contemplated. A washer-shaped retainer 28 is welded to rotary member 22 to secure mounting assembly 40 on shaft 22, as discussed in greater detail below. Opposite first end 26 is second end 30 of shaft 22. Second end 30 includes an integrally formed radially enlarged grip portion 32 and a threaded shaft portion 34. An axially extending cylindrical opening 36 defines a bore 38 on the distal face of second end 30.

As shown in Figures 1 and 3, mounting assembly 40 includes a mounting member 46 having a sleeve portion 48 and an integrally formed counterweight 50 and mounting stem 52. The mounting assembly 40 is preferably manufactured of stainless steel, but other materials are also contemplated as being within the scope of the invention. Sleeve portion 48 surrounds shaft 22 and defines a cylindrical opening 54 in which bearing sleeve 42 is located.
The radially distal end 56 of mounting stem 52 has a male dovetail joint 58 and a threaded opening 59 for mounting reference array 60 thereon.

Reference array 60 includes a support structure 62, which forms a female dovetail joint 64, and outwardly extending arms 68. The support structure 62 may be manufactured of aluminum, another metal, a plastic, or any other suitably rigid material. Each of the support arms 68 has a reference element 70 mounted thereon. At least one reference element 70 is included in the present invention, with four reference elements 70 being shown in the preferred embodiment. In the illustrated embodiment, reference elements 70 are reflective spheres which are registerable in a computer assisted navigation system, as discussed in greater detail below. A threaded fastener 66 passes through a hole 67 in support structure 62 and is securely engaged with threaded opening 59 to firmly secure array 60 on mounting stem 52 after engaging dovetail joints 58, 64. Of course, the support structure 62 may be attached to the mounting assembly 40 by means other than the dovetail joints and threaded fastener configuration shown in the figures, as long as the connection means chosen allows for a rigid connection between the two components.

A collet assembly 80 is located at second end 30 and is used to secure a rotating surgical tool, such as reamer 72, to shaft 22. Reamer 72 is a conventional reamer having a long shaft portion 74 with cutting threads and a blunt tip 76. Reamer 72 also includes a conventionally configured engagement shank 78. Collet assembly 80 includes a collet 82 having a small diameter portion 84 and a larger diameter portion 86. Collet 82 is preferably made of stainless steel, although other materials may also be used. In the illustrated embodiment, collet 82 includes four flexible fingers 88 which are separated by gaps 90 and may be biased radially inwardly into the central void space defined by collet 82. Gaps 90 extend centrally down fingers 88 and enhance the flexibility of collet 82. Additional gaps 94, which extend from small diameter portion 84 into the larger diameter portion 86, may also be provided to add extra flexibility to each of the fingers 88, if desired. A camming surface 92 is located at the distal ends of fingers 88 and is engageable with camming surface 98 of biasing member 100. Of course, it is contemplated that the number of flexible fingers 88 and gaps 90 could be varied, if desired.

A second camming surface 91 on collet fingers 88 engages the surface defining opening 38 when smaller diameter portion 84 of collet 82 is disposed into cylindrical bore 38. Larger diameter portion 86 extends outwardly from bore 36, and is circumscribed by biasing member 100. Biasing member 100 includes interior threads 102 which engage exterior threads 34. As biasing member 100 is increasingly engaged with threads 34, camming surface 98 biases collet fingers 88 radially inwardly and toward opening 36. Engagement of opening 36 with camming surfaces 91 also biases collet fingers 88 inwardly toward the central void defined by collet 82.

Collet assembly 80 may thereby firmly engage shank 78 of reamer 72 when it is inserted through opening 104 of biasing member 100. Collet fingers 88 may also be used to firmly grip other rotatable tools. Biasing member 100 also includes projections 106 disposed on opposite sides of opening 104 which engage flats 108 located on shank 78. Shank 78 preferably has a conventional configuration known as a Hudson connector/Trinkle adaptor. Collet fingers 88, however, may also be used with tools having alternative shaped shanks or engagement features.

An alternative second end 30a, which may be used instead of collet assembly 80, is shown in Figure 4. This alternative connector has an outer sleeve 110 which surrounds shaft 22a. Shaft 22a is similar to shaft 22 except for second end 30a. A biasing member 112 biases sleeve 110 in the direction indicated by arrow 109. The interior surface of sleeve 110 has two portions which have different diameters. Disengagement portion 114 has a larger diameter than locking portion 116. Both portions 114 and 116 face locking balls 118 disposed in openings in hollow cylindrical portion 120 of shaft 22a. When sleeve 110 is disposed in the position illustrated in Figure 4, balls 118 are biased inwardly by inner surface 116 of sleeve 110 and into engagement with a circumferentially extending depression 124 on shank 78 to thereby lock shank 78 within shaft 22a. Balls 118 secure shank 78 to shaft 22a, but do not prevent relative rotation of shank 78 with respect to shaft 22a. Projections 122 on shaft 22a engage flats 108 to prevent the relative rotation between shank 78 and shaft 22a. To dismount shank 78, sleeve 110 is moved in the direction indicated by arrow 111 and radially enlarged inner surface 114 allows locking balls 118 to disengage from shank 78.

The quick connect locking feature illustrated in Figure 4 includes four locking balls 118 to provide a relatively secure engagement between shaft 22a and the rotary tool engaged thereto. Manufacturing the quick connect fitting to relatively tight tolerances can also improve the engagement between the two shafts being joined. Collet assembly 80 located on shaft 22 also provides a relatively secure connection that maintains reamer 72 in a position in which its rotational axis is aligned with the axis 21 of shaft 22 and minimizes any movement of the rotational axis of reamer 72 relative to shaft 22 and mounting assembly 40, i.e., it inhibits wobbling of reamer 72.

By providing a relatively firmer connection between shaft 22, 22a and a rotating tool such as reamer 78, the tracking of the tool by a computer assisted navigational system may be improved by reducing the wobble of the tool relative to shaft 22, 22a. Oftentimes, conventional surgical drills have connections for engaging reamers or other rotating tools which allow some wobbling of the rotating tool. In such a situation, if a reference array were mounted to the housing of the drill, the position of the rotating tool calculated by the navigation system will be inaccurate to the extent that the tool wobbles and departs from its assumed position relative to the reference array which is directly tracked by the navigation system.

As can be seen in Figure 1, shaft 22 and mounting assembly 40 are positioned between drill 44 and reamer 78 and any wobble created by the connection between drill 44 and first end 26 of shaft 22 does not affect the relative position of reference array 60 and reamer 78. Reamer 78, or other rotatable tool, is firmly fixed to shaft 22 to prevent or minimize relative movement of the tool.

Mounting assembly 40 is provided to position array 60 and reference elements 70 mounted thereon at a predefined relative position to the attached tool so that a computer navigation system tracking the positions of reference elements 70 can determine the position of the tool attached to second end 30. The relative axial movement of array 60 and second end 30, and any tool secured thereto, is prevented by positioning mounting assembly 40 between grip 32 and retainer 28. When assembling together shaft 22 and mounting assembly 40, mounting assembly 40 is positioned on shaft 22 and then retainer 28 is welded to shaft 22 to secure mounting assembly 40 between grip 32 and retainer 28 and prevent relative axial displacement of mounting assembly 40 and shaft 22.

For navigation systems which require there to be a clear line of sight between the reference elements being tracked and the sensors tracking the elements, such as an optical system wherein the sensors detect light either reflected or emitted by the reference elements, it is desirable that the reference elements be positioned above axis 21 to increase their visibility. The navigation system may not recognize array 60 if it were position below axis 21 in an "upside down" orientation. Thus, it is generally desirable to position array 60 vertically above axis 21.

Reference numeral 51 indicates the location of the center of gravity of the mounting assembly 40 and is shown in Figure 5. Center of gravity 51 is for the entire mounting assembly 40 which rotates relative to shaft 22, and thus includes array 60. As can also be seen in Figure 5, mounting stem 52 is disposed diametrically opposite (with respect to axis 21) counterweight portion 50. As described above, reference array 60 is mounted on distal end 56 of mounting stem 52, which is located at a first angular position relative to axis 21. Center of gravity 51 defines a second angular position relative to axis 21 and, as shown by angle 53, the angular positions of the mounting point of array 60 and center of gravity 51 are separated by an angle of 180 degrees.

Because mounting assembly 40 is rotatable relative to shaft 22 and is not secured to any other part, gravitational forces acting on mounting assembly 40 will bias the center of gravity 51 of mounting assembly 40 toward a position directly below the rotational axis 21 when the rotational axis is generally horizontally disposed. The present invention utilizes a counterweight 50 that is radially spaced from axis 21 to control the position of center of gravity 51 of mounting assembly 40. Counterweight 50 is configured to position center of gravity 51 diametrically opposite array 60 and thereby gravitationally bias array 60 toward a position above axis 21. In the illustrated embodiment, mounting member 46, including counterweight portion 50, is preferably made of a relatively dense stainless steel, and array 60 is preferably made of a relatively light aluminum. Other materials, however, may also be used to position center of gravity 51 in a desired location. Stated in terms of angular position relative to axis 21, to maintain a reference element 70 at a position at or above axis 21 when axis 21 is generally horizontally disposed, the angular positions of the reference element and the center of gravity relative to axis 21 must be separated by at least 90 degrees.

By using two raised cylindrical portions 24 to engage bearing sleeve 42 proximate its ends, mounting member 46 is rotatably mounted on shaft 22 in a stable manner that limits the contact surface area between shaft 22 and bearing sleeve 42 to reduce frictional resistance to the relative rotation of shaft 22 and mounting assembly 40. In the illustrated embodiment, sleeve 42 is made of polytetrafluoroethylene (or PTFE, also known as Teflon@), however, metallic or other polymeric materials (such as polyetheretherketone (PEEK)) could also be used to form sleeve 42. Alternative bearings having different designs could also be positioned between shaft 22 and mounting member 46, or, shaft 22 could bear directly against mounting member 46.

Due to the presence of counterweight portion 50, array 60 will remain positioned above both shaft 22 and axis 21 as shaft 22 is rotated by drill 44 (or other rotary driver) and in turn rotates reamer 78 (or other rotary tool). Thus, the surgeon is not required to manually retain mounting assembly 40 in this desirable position. Counterweight 50 thereby acts as an anti-rotation feature on mounting assembly 40. An alternative embodiment of mounting assembly 40 could include an alternative anti-rotation feature such as an engagement arm adapted for engaging the housing of the drill or other non-rotating structure to prevent mounting assembly 40 from rotating with shaft 22. An advantage of counterweight 50 is that it provides an anti-rotation feature which is not dependent upon engagement with any other stationary structure. As used herein, an anti-rotation feature is a feature which inhibits the rotation of mounting assembly 40 about axis 21 relative to the surrounding environment but which still allows for the relative rotation of shaft 22 and mounting assembly 40.

As described above, array 60 is mounted on mounting arm 52 and includes four referencing elements 70. In the preferred embodiments, by providing at least three non-linearly positioned reference elements 70 on array 60, the determination of the position of these reference elements allows the computer assisted navigation system to calculate the position and orientation of reference array 60 and thereby also calculate the position and orientation of shaft 22 and a tool attached thereto. However, reference elements other than the type depicted in the preferred embodiments may also be used, whereby some such elements may only require a single element, or at least one element, as opposed to the at least three non-linearly positioned elements described above.

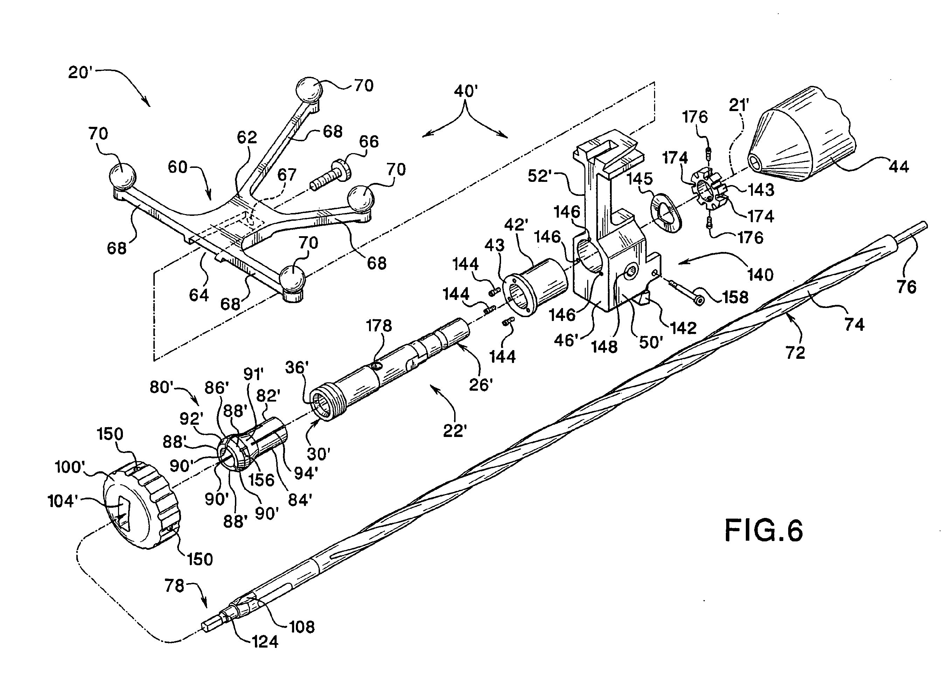

Turning now to Figures 6-8, a second embodiment of the surgical instrument of the present invention will be shown and described. Features of this embodiment that correspond to similar features of the other embodiment (shown in Figures 1-3 and 5) will be given the same reference numbers, except with the addition of the prime (') symbol. The second embodiment will be designated as surgical instrument 20', and it includes rotary member 22' that is rotationally engaged with mounting assembly 40'. The primary difference between this embodiment and the first embodiment is that this embodiment includes a locking assembly 140 with a locking lever 142 that interacts with a notched shaft retainer 143, which together provide a relatively easy means of selectively preventing relative rotation between rotary member 22' and mounting assembly 40'. In addition to the locking assembly, another benefit of this embodiment is that the assembly is axially shorter than the other embodiment, which potentially enables for more accurate location readings to be provided by the reference members 70 into the navigation system. Other differences and advantages of this embodiment will be described or will become apparent from the following description.

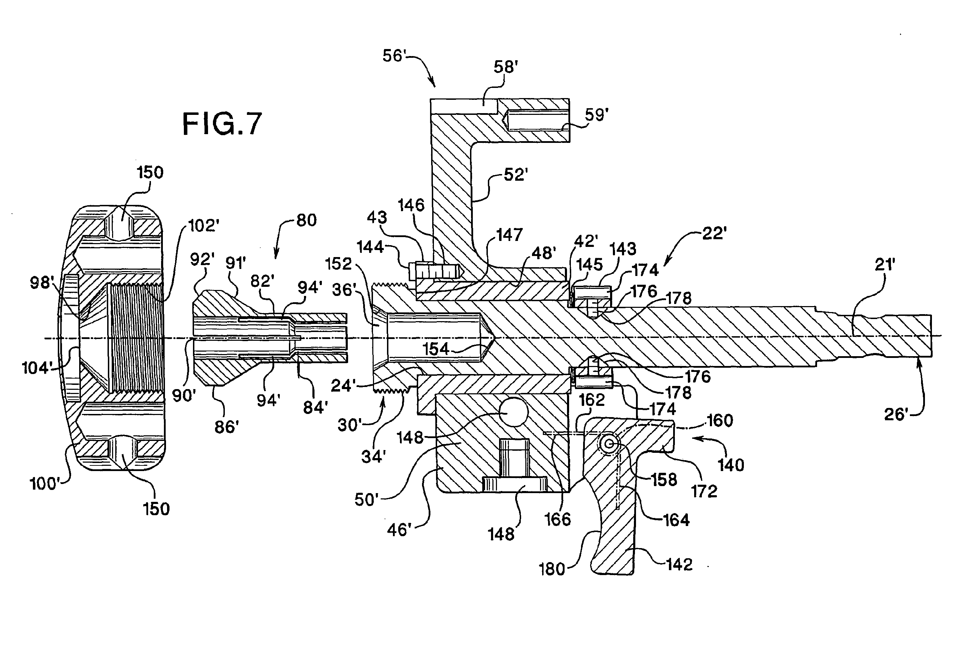

As with the first embodiment, the second embodiment includes a rotary member 22' that includes a first end 26' that is configured of a conventional shape to be inserted into, and rotated by, a surgical drill 44 (or other powered or manual rotary driver). Rotary member 22' further includes a cylindrical portion 24' that is configured to be seated within sleeve portion 48' of mounting member 46', with a bearing sleeve insert 42' therebetween, as shown in Figure 7. In the example of the second embodiment shown in Figure 7, cylindrical portion 24' is of a continuous uniform diameter. However, cylindrical portion 24' may also be broken up into two or more raised cylindrical portions (similar to portions 24 of the first embodiment, as shown in Figure 2) in order to reduce the frictional resistance, which may be necessary or desirable, depending upon the type of material used for sleeve insert 42'. Preferably, sleeve insert 42' is made from polyetheretherketone (PEEK), which should allow sleeve insert 42' to be made of a continuous diameter. Of course, the sleeve insert 42' could also be made of another polymeric material, such as polytetrafluoroethylene (or PTFE, also known as Teflon®), or of a metallic material, with the configuration of cylindrical portion 24' modified accordingly into two or more raised cylindrical portions. Sleeve insert 42' of this embodiment differs from sleeve insert 42 of the first embodiment because of the inclusion of shoulder 43, which has been provided to enable sleeve insert 42' to be attached to mounting member 46'. The illustrated example of this embodiment includes three screws 144 inserted into three apertures 146 formed in shoulder 43. However, a different number of screw/aperture configurations may also be used; the screw/aperture configurations may be omitted and another attachment means may be used; or the attachment feature may be omitted completely, if desired, and a non-attached bearing sleeve insert, similar to insert 42 of the first embodiment (shown in Figure 3) may be used, if desired.

The mounting member 46' of the second embodiment includes a mounting stem 52' with a distal end 56' of a similar configuration to that of the first embodiment so that the distal end 56' can be attached to reference array 60, which is the same as that described for the first embodiment. Since these features have been previously described with respect to the first embodiment, further description is unnecessary with regard to the second embodiment.

As with the first embodiment, a counterweight 50' is also provided as an integrally formed part of mounting member 46'. For either embodiment, it is also contemplated that the counterweight 50/50' could be provided as a separate component, with mounting member 46/46' acting as a housing for the counterweight.

Mounting member 46' also preferably includes at least one counter-bore 148, with three counter-bores 148 being included in the illustrated example of the second embodiment (with one counter-bore through the bottom and one through each side). Counter-bores 148 are each configured to accept a rod (not shown) to provide the user with better leverage for rotating mounting member 46' with respect to biasing member 100'. Similarly, biasing member 100' also preferably includes one or more bores 150 for the same purpose. In use, one rod is inserted into one of the counter-bores 148 and one rod is inserted into one of the bores 150, and the two rods are used to rotate mounting member 46 with respect to biasing member 100'. Multiple counter-bores 148 and multiple bores 150 are preferably provided, instead of just a single one of each, to provide unhindered, convenient access to at least one bore and at least one counter-bore, regardless of the manner in which surgical instrument 20' is positioned. Similar features may also be provided on the first embodiment, if desired.

A collet assembly 80' to secure a rotating surgical tool, such as reamer 72 (or any other rotating surgical tool), to rotary member 22' is also provided at the second end 30' of rotary member 22' of the second embodiment. Collet assembly 80' is of a somewhat different configuration from that of the first embodiment. More specifically, collet assembly 80' includes collet 82', biasing member 10' and opening 36' in second end 30' of rotary member 22', wherein all three of these features differ, at least slightly, from similar features of the first embodiment.

Collet 82' preferably includes a small diameter portion 84' and a larger diameter portion 86', which are each preferably divided into a plurality of flexible fingers. More specifically, gaps 90', which extend from the end near the larger diameter portion 86', divide collet 82' into a plurality of flexible fingers 88', with three fingers 88' being shown in this example. Preferably, collet 82' also includes at least one secondary gap 94' that extends from the smaller diameter end 84' and into the larger diameter end 86'. One or more secondary gaps 94' may optionally be provided in one or in all of the fingers 88' to add extra flexibility, with the preferred embodiment including one secondary gap 94' in each finger 88'.

Like the first embodiment, collet 82' of the second embodiment includes a first camming surface 92' and a second camming surface 91', with the preferred configurations of the camming surfaces of the second embodiment being different from those of the first embodiment. More specifically, while both camming surfaces 91 and 92 of the first embodiment are essentially the same size (both axially and radially), camming surface 91' of the second embodiment is larger, both axially and radially, than camming surface 92'. The larger camming surface 91' allows better interaction with tapered camming surface 152, which is included in this embodiment at one end of opening 36 on rotary member 22'. An additional camming surface, surface 98', is also provided on biasing member 100' for interacting with camming surface 92'. Of course, variations in the configurations of the camming surfaces are contemplated as being within the scope of the invention.

Biasing member 100' preferably includes interior threads 102' that engage with exterior threads 34' on second end 30' of rotary member 22'. As biasing member 100' is tightened against second end 30' of rotary member 22', camming surface 98' engages camming surface 92', thereby biasing fingers 88' radially inwardly. Fingers 88' are also biased radially inwardly when camming surface 91' engages camming surface 152, as biasing member 100' is further tightened. In this embodiment, opening 36' preferably includes a tapered end portion 154, which acts as another camming surface for biasing small diameter portion 84' of collet 82 radially inwardly. Preferably, at least one notch 156 (and more preferably, one notch on each finger) is also provided on larger diameter portion 86' for facilitating more uniform reduction in diameter when collet 82' is compressed when biasing member 100' is tightened against threaded shaft portion 34' of rotary shaft 22'. Through the use of multiple camming surfaces and flexible fingers, the collet assembly 80' thereby firmly engages shank 78 of reamer 72 (or other rotary tool) when shank 78 is inserted through opening 104' and biasing member 100' is tightened. Although collet assembly 80' has been shown and described, it is also contemplated that other types of shank connector mechanisms, such as alternative second end 30a shown in Figure 4 or collet assembly 80 of the first embodiment or another mechanism completely, may be used in the second embodiment instead of collet assembly 80'.

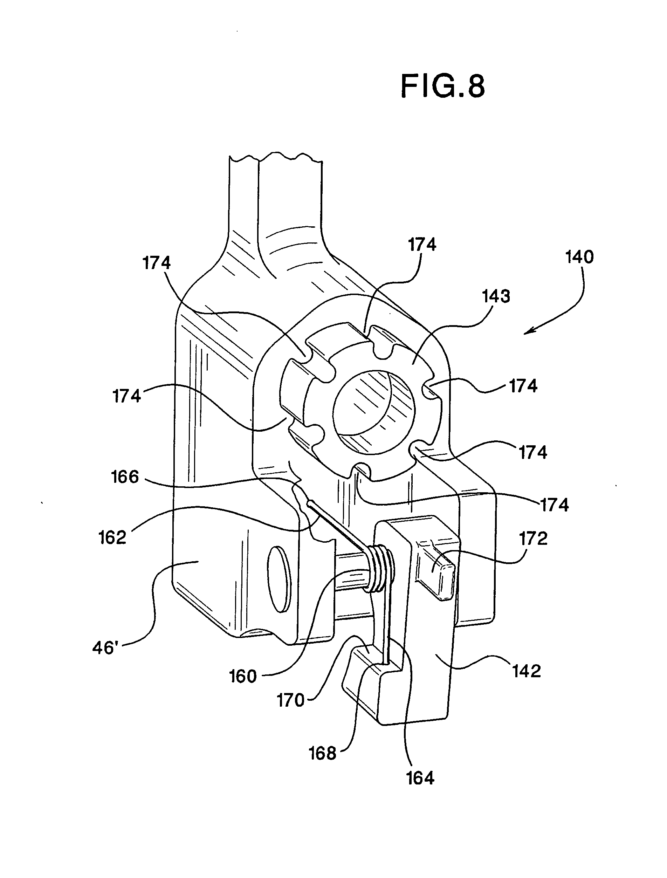

Turning now to Figures 7 and 8 of the locking assembly 140 will now be described (where Figure 8 is an end view with rotary member 22' removed and that has been partially cut-away to better show spring member 160). Locking assembly 140 is provided for temporarily locking mounting assembly 40' with respect to rotary member 22' to prevent relative rotation therebetween, such as when biasing member 100' is being threaded upon (or unthreaded from) second end 30' of rotary member 22'. As mentioned earlier, locking assembly 140 includes locking lever 142, which is pivotably mounted on lever screw 158. Locking lever 142 is biased into the closed position, which is the position shown in Figure 7, by spring member 160, which is preferably a generally "L" shaped torsion spring that includes legs 162 and 164. Leg 162 is preferably maintained in position by being inserted into an aperture 166 in counterweight 150', and leg 164 is preferably maintained in position by being inserted into aperture 168, which, as seen in Figure 8, is provided within a step portion 170 of locking lever 142. Instead of "L" shaped torsion spring 160, another form of biasing means could be used instead, such as an appropriately placed coil spring.

Locking lever 142 also includes at least one projection 172 that is configured to mate with at least one notch 174 provided on a shaft retainer 143. Shaft retainer 143 may include only a single notch, but it preferably includes a plurality of notches to avoid requiring rotation of shaft retainer 143 with respect to locking lever 142 so that lever 142 can mate with a notch. Further, it should be noted that the locations of the projections and the notches could be reversed, such that the shaft retainer 143 includes one or more projections and the locking lever includes at least one notch.

Shaft retainer 143 is rigidly mounted to rotary member 22' for rotation therewith. Although various methods of mounting shaft retainer 143 to member 22' may be used (such as a keyed arrangement or a set of corresponding non-circular cross-sections), the illustrated example of the second embodiment utilizes a setscrew configuration. More specifically, shaft retainer 143 is preferably rigidly affixed to rotary member 22' by at least one setscrew 176 (with two set screws being used in the preferred embodiment) inserted into a threaded aperture in shaft retainer 143. Preferably setscrew 176 engages a depression 178 formed in rotary shaft 22'. The setscrew(s) 176 should not interfere with the notches 174, so if they are located within the notches 174, the setscrews should be short enough for their tops to be seated flush with the bottom of the notch, as shown in Figure 7. Otherwise, the setscrews can be positioned at positions radially away from the notches 174. Although depressions 178 provide for a more secure attachment arrangement, the depressions may be omitted if desired.

In addition to providing the notches for the locking assembly, shaft retainer 143 also functions to maintain rotary member 22' in position axially. More specifically, shaft retainer 143 cooperates with shoulder 147 (on second end 30') to prevent rotary shaft member 22' from moving axially out of engagement with sleeve portion 48' (via bearing sleeve insert 42'). If desired, a wave washer 145 may be placed between shaft retainer 143 and bearing sleeve insert 42' to provide some axial play.

In order to engage the locking assembly 140 to prevent relative rotation between rotary member 22' and mounting member 46', such as when one intends to tighten (or loosen) biasing member 100' against threaded shaft portion 34', locking lever 142 is pushed in the counter-clockwise direction (with respect to the Figure 7 view), i.e., against the biasing force of spring member 160, until projection 172 mates with one of the notches 174. For the user's comfort, a curved surface 180 is preferably provided on locking lever 142, whereby the user's finger can comfortably engage curved surface 180 of lever 142, like a trigger, in order to engage the locking assembly 140 and to maintain it in a locked position. When the user wants to disengage the locking assembly 140 from the locked position, the user simply releases the pressure applied by his/her finger upon curved surface 180 of lever 142, and spring member 160 biases lever 142 so that projection 172 is no longer in engagement with one of the notches 174. At which point, if biasing member 100' is tightened and shank 78 is firmly engaged, the rotary tool may be used and the counterweight 50' will operate in the same manner as in the first embodiment to maintain array 60 vertically above axis 21'.

The axial length of the assembly of the second embodiment (Figures 6-8) is reduced when compared to that of the first embodiment (Figures 1-5,) because grip portion 32 of the first embodiment has been eliminated, which allows rotary member 22' of the second embodiment to be of a shorter axial length when compared to rotary member 22 or 22a of the first embodiment. Also, some of the components are also made of a reduced axial length, such as biasing member 100', collet 82' and mounting member 46'. Such a reduced axial length allows for better accuracy of the navigation system.

As is known in the art, data concerning the fixed size and shape of a surgical instrument, such as reamer 78, which will be used in an image guided procedure can be determined pre-operatively to obtain a three dimensional model of the instrument or the relevant portions thereof. Additionally, the relevant dimensional data concerning an anatomical structure of interest, e.g., a femur, may be determined using data acquired from images of the anatomical structure to generate a data base representing a model of the anatomical structure. The model of the anatomical structure may be a three dimensional model which is developed by acquiring a series of two dimensional images of the anatomical structure. Alternatively, the model of the anatomical structure may be a set of two dimensional images having known spatial relationships or other data structure which can be used to convey information concerning the three dimensional form of the anatomical structure. The model of the anatomical structure may then be used to generate displays of the anatomical structure from various perspectives for preoperative planning purposes and intraoperative navigational purposes. A variety of technologies which may be employed to generate such a model of an anatomical structure are well known in the art and include computed tomography (CT), magnetic resonance imaging (MRI), positron emission tomography (PET), ultrasound scanning and fluoroscopic imaging technologies.

The model of the anatomical structure obtained by such imaging technologies can be used for the intraoperative guidance of a surgical instrument by facilitating the determination and display of the relative position and orientation of the surgical instrument with respect to the actual anatomical structure. For example, if the model of the anatomical structure is a set of two dimensional images having known spatial relationships, several such images may be simultaneously displayed during the surgical procedure. By also displaying the position of the surgical instrument in the images and displaying images taken from different perspectives, e.g., one image facilitating the display of instrument movement along the x and y coordinate axes and another image facilitating the display of instrument movement along the z axis, the individual images may together represent the movement of the surgical instrument in three dimensions relative to the anatomical structure.

For reference purposes, a coordinate system defined by the actual anatomical structure which is the subject of interest will be referred to herein as the anatomical coordinate system and a coordinate system defined by the model of the anatomical structure will be referred to as the image coordinate system.

Rigid anatomical structures, such as skeletal elements, are well suited for such image guided surgical techniques and individual skeletal elements may be used to define separate coordinate systems. The different rigid structures, e.g., skeletal elements, may be subject to relative movement, for example, the femur and acetabulum of a patient may be relatively moved during the surgical procedure and separate three dimensional models and coordinate systems may be created for the different skeletal elements. For example, during a hip replacement procedure, a three dimensional model of the femur defining a first coordinate system may be utilized during the preparation of the femur while a separate coordinate system defined by a three dimension model of the pelvis may be utilized during the preparation of the acetabulum.

When using computer assisted navigation, also referred to as computer implemented image guidance, to conduct a surgical technique, the image coordinate system is registered with the anatomical coordinate system and the position of the surgical instrument or other tracked object is also registered within the image coordinate system. After the registration of both the actual anatomical structure and the surgical instrument, the relative position and orientation of the surgical instrument may be communicated to the surgeon by displaying together images of the anatomical structure and the instrument based upon the three dimensional models of the anatomical structure and instrument which were previously acquired.

Instruments registerable within a computer assisted navigation system and which could be employed or adapted for use as digitizing probes to engage a tool at a known location, such as tip 76 of reamer 72, and thereby calibrate the position of tip 76 relative to array 60 in the navigational system are described by Grimm et al. in a U.S. Patent Application entitled IMPLANT REGISTRATION DEVICE FOR SURGICAL NAVIGATION SYSTEM having Serial No. 10/357,754, filed on February 4, 2004, and by McGinley et al. in a U.S. Patent Application entitled SURGICAL NAVIGATION INSTRUMENT USEFUL IN MARKING ANATOMICAL STRUCTURES having Serial No. 10/357,959, filed on February 4, 2003, and the disclosures of both of these applications are hereby incorporated herein by reference.

Computer implemented image guidance systems which provide for the registration of an actual anatomical structure with a three dimensional model representing that structure together with the registration or localization of another object such as a surgical instrument or orthopedic implant within the image coordinate system to facilitate the display of the relative positions of the object and the actual anatomical structure are known in the art. Known methods of registering the anatomical structure with the image coordinate system include the use of implanted fiducial markers which are recognizable by one or more scanning technologies. Alternatively, implants may be located by physically positioning a digitizing probe or similar device in contact or at a known orientation with respect to the implant. Instead of using fiducial implants, it may also be possible to register the two coordinate systems by aligning anatomical landmark features. U.S. Patent Nos. 6,236,875 B1 and 6,167,145 both describe methods of registering multiple rigid bodies and displaying the relative positions thereof and the disclosures of both of these patents are hereby incorporated herein by reference.

Tracking devices employing various technologies enabling the registration or localization of a surgical instrument and the tracking of the instrument motion with respect to the anatomical coordinate system, which has also been registered with the image coordinate system, are also known. For example, optical tracking systems which detect light reflected from or emitted by reflective targets or localizing emitters secured in a known orientation to the instrument are known for determining the position of the instrument and registering the position of the instrument within an image coordinate system representing a three dimensional model of an anatomical structure. For example, such a tracking system may take the form of a sensor unit having one or more lenses each focusing on a separate charge coupled device (CCD) sensitive to infrared light. The sensor unit detects infrared light emitted by three or more non-linearly positioned light emitting diodes (LEDs) secured relative to the object. A processor analyzes the images captured by the sensor unit and calculates the position and orientation of the instrument. By registering the position of the sensing unit within the image coordinate system, the position of the instrument relative to the anatomical structure, which has also been registered with the image coordinate system, may be determined and tracked as the instrument is moved relative to the anatomical structure.

Alternative localizing systems may employ localizing emitters which emit an electromagnetic signal in the radio frequency or which emit visible light. Other types of localizing systems that could be used with the present invention employ referencing elements or other distinguishing elements which are radio-opaque. It is also possible to employ digitizing physical probes which are brought into physical contact with the object at predefined locations on the object to register the position of the object.

In the disclosed embodiments, the localizing system includes a light source and reference elements 70 reflect the light. The localizing system then detects the reflected light and computes the location of the individual reference elements 70 in a known manner. Reference elements 70 may be obtained from Northern Digital Inc. having a place of business at 103 Randall Dr., Waterloo, Onterio, Canada, N2V1C5. Northern Digital Inc. supplies image guidance systems under the brand names Optotrak® and Polaris® which may be used with the present invention. The present invention may also be used with other computer assisted navigation systems such as those described above or otherwise known in the art. For example, Medtronic, Inc. headquartered in Minneapolis, Minnesota, manufactures and sells various computer assisted surgical navigation systems under the trademark StealthStation®, such as the FluoroNav™ Virtual Fluoroscopy System, which could also be adapted for use with the present invention.

An alternative embodiment of the present invention could be employed with a computer assisted navigation system which utilizes magnetic fields, instead of optical tracking, to determine the position and orientation of the tracked object. A variety of referencing elements which are used with magnetic fields which could be adapted for use with the present invention are known in the art. For example, known systems using magnetic fields to determine the position and orientation of an object are described by U.S. Pat. Nos. 5,913,820; 6,381,485 B1; 6,402,762 B2; 6,474,341 B1; 6,493,573 B1; and 6,499,488 B1, and the disclosures of these patents are all hereby incorporated herein by reference.

By generating a magnetic field of known properties in the operative area and sensing the field with mutually perpendicular wire loops, the position and orientation of the reference elements defined by the wire loops and the rigid object, such as a surgical instrument, attached thereto may be calculated. The determination of the position and orientation of such mutually perpendicularly oriented field sensors is known in the art. It is also known to use a single wire loop to form a single field sensor and determine its position and orientation by generating magnetic fields from a plurality of locations.

While this invention has been described as having exemplary designs, the present invention may be further modified within the spirit and scope of this disclosure. This application is therefore intended to cover any variations, uses, or adaptations of the invention using its general principles. The following paragraphs indicate variations on the invention..
1.A surgical instrument for use in a computer assisted navigation system, said instrument comprising:
   a rotary member having first and second opposed ends;
   a mounting assembly operably coupled to said rotary member wherein said rotary member and said mounting assembly are relatively rotatable about an axis; and
   at least one reference element registerable in the computer assisted navigation system disposed on said mounting assembly at a predetermined location, said mounting assembly defining a center of gravity spaced radially outwardly from said axis wherein said predetermined location defines a first angular position relative to said axis and said center of gravity defines a second angular position relative to said axis, said first and second angular positions separated by at least 90 degrees.
2. The surgical instrument of paragraph 1 wherein said mounting assembly comprises a sleeve portion defining a cylindrical opening, said rotary member rotationally disposed within said cylindrical opening, said mounting assembly having a counterweight portion disposed radially outwardly from said axis, said at least one reference element disposed substantially diametrically opposite said counterweight portion relative to said axis.
3. The surgical instrument of paragraph 2 wherein said mounting assembly further includes a mounting stem extending radially outwardly from said sleeve portion and disposed substantially diametrically opposite said counterweight portion relative to said axis, said at least one reference element being positioned at a radially distal end of said mounting stem.
4. The surgical instrument of paragraph 3 wherein said rotary member includes a cylindrical shaft portion rotatably engaged with said mounting assembly.
5. The surgical instrument of paragraph 4, further comprising a bearing sleeve insert positioned between said cylindrical shaft portion of said rotary member and said mounting assembly.
6. The surgical instrument of paragraph 5 further comprising a rotational driver detachably securable to said first end; and
   a rotatable tool detachably securable to said second end.
7. The surgical instrument of paragraph 6 wherein said rotary member further comprises a collet assembly disposed at said second end, said collet assembly including a collet and a biasing member, said collet defining a central void and having a plurality of fingers biasable inwardly relative to said void, said biasing member biasingly engageable with said plurality of collet fingers and wherein said biasing member is securable in a position biasing said plurality of fingers inwardly relative to said central void.
8. The surgical instrument of paragraph 7 further comprising a surgical tool having a shank, said shank being insertable into said central void, said shank being rotationally fixedly engageable by said inwardly biasable plurality of collet fingers.
9. The surgical instrument of paragraph 1 wherein said second end is defined by a cylindrical shaft having exterior threads and an axially disposed opening, wherein said collet is partially positioned in said opening, with at least a portion of said plurality of collet fingers projecting from said opening, and said biasing member is threadingly engaged with said exterior threads and circumscribes said projecting portion of said plurality of collet fingers.
10. The surgical instrument of paragraph 1, further comprising a locking assembly for temporarily locking said mounting assembly with respect to said rotary member to prevent relative rotation therebetween.
11. The surgical instrument of paragraph 1, wherein said locking assembly includes:
   a shaft retainer positioned on said rotary member for rotation therewith; and
   a locking lever pivotably attached to said mounting assembly, whereby said locking lever is configured to be pivoted to engage a portion of said shaft retainer, thereby preventing relative rotation between said rotary member and said mounting assembly.
12. A surgical instrument for use in a computer assisted navigation system, said instrument comprising:
   a rotary member;
   a mounting assembly operably coupled to said rotary member wherein said rotary member and said mounting assembly are relatively rotatable about an axis;
   at least one reference element registerable in the computer assisted navigation system, said at least one reference element disposed on said mounting assembly at a predetermined location; and
   an anti-rotation feature disposed on said mounting assembly biasing said mounting assembly toward an orientation wherein said reference element is disposed vertically above said axis during relative rotation of said rotary member and said mounting assembly about said axis when said axis is generally horizontally disposed.
13. The surgical instrument of paragraph 12 wherein said anti-rotation feature comprises a counterweight secured to said mounting assembly diametrically opposite said at least one reference element relative to said axis.
14. The surgical instrument of paragraph 12 further comprising a collet assembly disposed at one end of said rotary member, said collet assembly including a collet and a biasing member, said collet defining a central void and having a plurality of fingers biasable inwardly relative to said void, said biasing member biasingly engageable with said plurality of collet fingers and wherein said biasing member is securable in a position biasing said plurality of fingers inwardly relative to said central void.
15. The surgical instrument of paragraph 12, further comprising a locking assembly for temporarily locking said mounting assembly with respect to said rotary member to prevent relative rotation therebetween.
16. The surgical instrument of paragraph 12, wherein said locking assembly includes:
   a shaft retainer positioned on said rotary member for rotation therewith; and
   a locking lever pivotably attached to said mounting assembly, whereby said locking lever is configured to be pivoted to engage a portion of said shaft retainer, thereby preventing relative rotation between said rotary member and said mounting assembly.
17. The surgical instrument of paragraph 12., wherein said locking assembly further includes:
   at least one notch on one of said shaft retainer or said locking lever;
   at least one projection on the other of said shaft retainer or said locking lever, wherein said at least one projection is configured to mate with said at least one notch for preventing relative rotation between said rotary member and said mounting assembly; and
   a spring member configured to bias said locking lever out of engagement with said shaft retainer.

## Claims

1. A surgical instrument for use in a computer assisted navigation system, said instrument comprising:
at least one reference element registerable in the computer assisted navigation system;
a mounting assembly defining an axis and rotatably mounted on the instrument, said at least one reference element positionable on said mounting assembly in a predetermined location, said predetermined location defining a first angular position relative to said axis; and
a counterweight disposed on said mounting assembly and radially outwardly spaced from said axis, said counterweight defining a second angular position relative to said axis, said first and second angular positions separated by at least 90 degrees.

2. The surgical instrument of claim 1 wherein said mounting assembly further comprises a radially outwardly extending mounting stem, said stem disposed substantially diametrically opposite said counterweight relative to said axis, said at least one reference element being positioned at a radially distal end of said mounting stem.

3. The surgical instrument of claim 1 wherein said mounting assembly includes a sleeve portion defining a cylindrical opening and said counterweight is integrally formed with said sleeve portion.

4. The surgical instrument of claim 3 further comprising a rotary member having a cylindrical shaft portion rotationally engaged with said sleeve portion of said mounting assembly.

5. The surgical instrument of claim 4, further comprising a bearing sleeve insert positioned between said cylindrical shaft portion of said rotary member and said sleeve portion of said mounting assembly.

6. The surgical instrument of claim 4, further comprising a collet assembly disposed at an end of said rotary member, said collet assembly including a collet and a biasing member, said collet defining a central void and having a plurality of fingers biasable inwardly relative to said void, said biasing member biasingly engageable with said plurality of collet fingers and wherein said biasing member is securable in a position biasing said plurality of fingers inwardly relative to said central void.

7. The surgical instrument of claim 6 wherein said end of said rotary member is defined by a cylindrical shaft having exterior threads and an axially disposed opening, wherein said collet is partially positioned in said opening, with at least a portion of said plurality of collet fingers projecting from said opening, and said biasing member is threadingly engaged with said exterior threads and circumscribes said projecting portion of said plurality of collet fingers.

8. The surgical instrument of claim 1, further comprising:
a rotary member having a cylindrical shaft portion engaged for relative rotation with respect to a sleeve portion of said mounting assembly; and
a locking assembly for temporarily locking said mounting assembly with respect to said rotary member to prevent relative rotation therebetween.

9. The surgical instrument of claim 8, wherein said locking assembly includes:
a shaft retainer positioned on said rotary member for rotation therewith; and
a locking lever pivotably attached to said mounting assembly, whereby said locking lever is configured to be pivoted to engage a portion of said shaft retainer, thereby preventing relative rotation between said rotary member and said mounting assembly.

10. The surgical instrument of Claim 9, wherein said locking assembly further includes:
at least one notch on one of said shaft retainer or said locking lever; and
at least one projection on the other of said shaft retainer or said locking lever, wherein said at least one projection is configured to mate with said at least one notch for preventing relative rotation between said rotary member and said mounting assembly.
